# EUROPEAN PATENT APPLICATION

(11) **EP 2 853 260 A1**
(43) Date of publication of application: **01.04.2015**
(21) Application number: 13186353.2
(22) Date of filing: 27.09.2013
(51) Int. Cl.: A61K 9/20, A61K 31/4439

(54) **Pharmaceutical preparation comprising dabigatran etexilate bismesylate**

(71) Applicant: ratiopharm GmbH, 89079 Ulm (DE)
(72) Inventor: Ackermann, Dorothee, 81379 München (DE); Leutner, Dirk, 80939 München (DE); Mika, Hans Juergen, 53229 Bonn (DE)
(74) Representative: Teipel, Stephan

(57) **Abstract**

The present invention relates to a pharmaceutical preparation comprising dabigatran etexilate bismesylate.

## Description

The present invention relates to a pharmaceutical preparation comprising dabigatran etexilate bismesylate.

Dabigatran etexilate (ethyl 3-{[(2-{[(4-{N'-[(hexyloxy)carbonyl]carbamimidoyl}phenyl)amino] methyl}-1-methyl-1H-benzimidazol-5-yl)carbonyl](2-pyridinyl)amino}propanoate) has the following chemical structure (I):

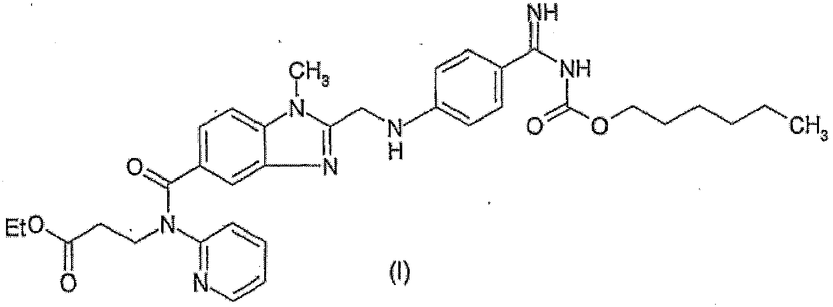

This pharmaceutically active ingredient is known from WO 98/37075. Dabigatran is an anticoagulant from the class of the direct thrombin inhibitors. It is orally administered as the prodrug dabigatran etexilate which is marketed as Pradaxa^{®} in European countries. It is used in the primary prevention of venous thromboembolic events (primary VTE prevention) in post-operative orthopedic adult patients and in the prevention of stroke and systemic embolism in adult patients with nonvalvular atrial fibrillation (AF).

Dabigatran etexilate has a solubility in water of only 1.8 mg/ml. Moreover, the solubility strongly depends on the pH. In acid environment the solubility is strongly increased. This results in problems in the bioavailability of the active ingredient because its solubility depends on the pH value in the gastrointestinal tract, such as in the stomach of the patient. Particular problems arise for patients having a stomach wherein the pH value is altered, for example due to physiolgical variabiliy, diseases or promedication, such as due to PP inhibitors.

The problems associated with the pH-dependent solubility are dealt with in WO 03/074056 and WO 2005/018615. Both documents suggest combining dabigatran etexilate or a pharmaceuticalla acceptable salt thereof and an organic acid having a certain solubility within a pharmaceutical composition. It is said that due to this combination the bioavailability of the active agent is substantially independent of the pH in the stomach. As salt of dabigatran etexilate only dabigatran etexilate monomesylate is suggested. Furthermore, WO 03/074056 suggests incorporating the organic acid in a core material and separating the active substance layer and the core material from one another by an insulating layer consisting of a water-soluble polymer in order to increase the storage stability of the product.

A pharmaceutical controlled release system for administration of active substances with pH-dependent solubilities, such as, among others, dabigatran etexilate comprising a core material comprising a pH modifier, such as an organic acid, and a layer comprising the active substance which are separated from each other by a layer comprising a water-insoluble polymer is disclosed in WO 2008/022932.

The pellet formulation used in the marketed product Pradaxa^{®} and described in WO 03/074056 is produced by a difficult production method. For pellet processing specially designed production equipment has to be used. The process is also time and energy consuming due to the time required for applying the different layers and the energy required for the fluid bed coater for generating the fluid bed and heating of the air stream.

If the preparation comprising an organic acid is not prepared in the form of pellets having several layers separating the organic acid from the active ingredient but in the form of usual tablets as described in WO 2005/018615 it was found by the present inventors that these tablets do not show any long-term stability. The dabigatran etexilate degrades extensively, especially when adding tartaric acid as used in the already marketed product. Also high degradation was found with fumaric acid, which is used in the examples of WO 2005/018615. It was found that the active ingredient degrades to an extent that these formulations are not applicable for the production of a stable, marketable product.

There is therefore still a need for pharmaceutical preparations which contain dabigatran etexilate and which release the active ingredient less dependent of the pH but which are nevertheless sufficiently stable for usual storage and which can easily be prepared without requiring difficult and time consuming preparation methods.

It has now surprisingly been found that the above described problems are solved by a pharmaceutical preparation comprising a specific salt of dabigatran etexilate, namely dabigatran etexilate bismesylate. In this case, the preparation can be prepared without any organic acid as pH modifier. Therefore, the stability problems associated with the prior art preparations and the difficulties associated with the prior art preparation methods are overcome. This finding is particularly surprising because compared to the dabigatran etexilate monomesylate salt used in the prior art preparations, dabigatran etexilate bismesylate contains an additional molecule of an organic acid within the drug salt itself. In view of the known instability of the monomesylate salt in the presence of organic acids, it was expected that the bismesylate salt is more likely to be instable in formulations compared to formulations using an externally added organic acid. This is because the organic acid component which is inducing instability is within the bismesylate salt form in even closer contact to the dabigatran etexilate molecule compared to blends with externally added organic acids. As will be shown in the examples below, this is even valid for formulations with an equimolar amount of tartaric acid as external excipient compared to the equimolar amount of methane sulphonic acid in the salt form of the drug.

The present invention therefore relates to a pharmaceutical preparation comprising dabigatran etexilate bismesylate and at least one pharmaceutically acceptable excipient, characterized in that the preparation does not contain any organic acid having a solubility of > 1 g/250 ml of water at 20°C.

Compared to stability tests of the prior art formulations the invention surprisingly creates stable pharmaceutical preparations and in particular tablets and capsules which can be used for marketable products avoiding the costly production method of pellet layering required in the prior art. Due to the surprising stability of the pharmaceutical preparations of the present invention, the production method is eased extensively compared to the already known pellet process. This helps the manufacturer to produce drug formulations most effectively by using for example direct compression or standard granulation methods before capsule filling. Besides reducing production costs, the invention relates to eased production of solid dosage forms with different release profiles.

In the pharmaceutical preparation of the present invention the dabigatran etexilate is sufficiently stable for usual storage of pharmaceuticals. In this regard "stable" relates to the chemical stability of the active ingredient. In particular, the active ingredient is stable without the requirement of separating the active ingredient from other ingredients of the preparation which therefore is easier in manufacture. The pharmaceutical preparation of the invention nevertheless exhibits good solubility and bioavailability and in particular provides a pH-independent bioavailability without the requirement of any organic or other acid being present in the preparation as additional component.

A particular advantage of the present invention is that the pharmaceutical preparation.may contain the active ingredient in an admixture with all other excipients (except optional coatings) without the requirement of separating the active ingredient from certain excipients, for example in different layers or compartments.

Preferably, the pharmaceutical preparation does not contain any organic acid exhibiting a pH value in water of below 6 at a concentration of 1 part by weight of the acidic excipient per 100 parts by weight of water. In a further preferred embodiment the pharmaceutical preparation additionally does not contain any inorganic acid exhibiting a pH value in water of below 6 at a concentration of 1 part by weight of the inorganic acid per 100 parts by weight of water. In both cases the pH is measured at 23°C after dissolving the acid in the water or, if the acid does not completely dissolve in the water, after stirring the dispersion of the acid in the water for 10 minutes.

In a further embodiment the organic or inorganic acid, which is not present in the pharmaceutical preparation according to the invention, has a pkₐ₁ value below 5. Even more preferably the pharmaceutical preparation does not contain any organic or inorganic acid. Most preferably the pharmaceutical preparation does not contain any acidic excipient.

Examples of organic and inorganic acids which are not contained within the pharmaceutical preparation are tartaric acid, fumaric acid, succinic acid, citric acid, malic acid, glutaric acid, aspartic acid, hydrochloric acid, sulphuric acid, phosphonic acid, or hydrates or acid salts thereof.

The amount of dabigatran etexilate bismesylate in the pharmaceutical preparation of the present invention is not particularly limited. The amount can be, for example, in the range of 10 to 90 % by weight, such as above 20 % by weight, above 35 % by weight, above 40 % or above 45 % by weight dabigatran etexilate bismesylate based on the total weight of the composition. Suitably, a unit dose of the pharmaceutical preparation, such as a tablet or capsule, contains 25 to 300 mg of dabigatran etexilate calculated as free base. If the pharmaceutical preparation is present as immediate release preparation, the unit dose may for example contain 50 to 150 mg dabigatran etexilate calculated as free base and, if the pharmaceutical preparation is an extended release preparation a unit dose may for example contain 100 to 300 mg dabigatran etexilate calculated as free base.

In one embodiment, the dabigatran etexilate bismesylate employed in the pharmaceutical preparation according to the invention can be crystalline. Crystalline dabigatran etexilate bismesylate can be obtained as described in WO 2012/044595. The crystalline polymorphic form called "Ms2-A" of dabigatran etexilate bismesylate as disclosed in this document is particularly preferred.

The pharmaceutical preparation of the invention can be an oral solid preparation, such as a powder mixture, tablet, capsule, pellets or granules. Such pharmaceutical dosage forms can be prepared by methods known in the art, such as tabletting by granulation or direct compression. For example, granules being obtained by usual wet or dry granulation methods can be compressed into tablets, filled into hard capsules, sachets, stick packs or processed into Multi-Unit Pharmaceutical Systems (MUPS).

Another advantage of the present invention is that the excipients can be easily selected such as to tailor the release profile of the pharmaceutical preparation without requiring complicated layer systems as described for example in WO 2008/022932. For example, the ingredients can be selected in order to prepare a dosage form having an immediate-release profile or a modified-release profile, such as an extended-release profile.

For example, the pharmaceutical preparation may contain the active ingredient within a matrix forming excipient, thus forming for example an immediate-release matrix or an extended-release matrix. The release profile may additionally or alternatively be adjusted by one or more coatings and/or an osmotic device as known to the skilled person.

Within the present application generally the term "modified release" is used as defined by the USP. Preferably, modified release dosage forms are those whose drug release characteristics accomplish therapeutic or convenience objectives not offered by immediate release forms. Generally, immediate release (IR) forms release at least 70 % of the drug within 1 hour or less. The term "modified release" can comprise delayed release, prolonged release, sustained release, extended release and/or controlled release.

Delayed release usually indicates that the drug (i.e., dabigatran etexilate) is not being released immediately after administration but at a later time, preferably less than 10 % are released within two hours after administration.

Prolonged release usually indicates that the drug (i.e., dabigatran etexilate) is provided for absorption over a longer period of time than IR forms, preferably for about 2 to 24 hours, in particular for 4 to 16 hours.

Sustained release usually indicates an initial release of drug (i.e. dabigatran etexilate), sufficient to provide a therapeutic dose soon after administration, preferably within two hours after administration, and then a gradual release after an extended period of time, preferably for about 3 to 18 hours, in particular for 4 to 12 hours.

Extended release (ER) usually indicates a slow drug (i.e., dabigatran etexilate) release, so that plasma concentrations are maintained at a therapeutic level for a time period of between 6 and 36 hours, preferably between 8 and 24 hours.

Controlled release dosage forms usually release the drug (i.e., dabigatran etexilate) at a constant rate and provide plasma concentrations that remain essentially invariant with time.

In a preferred embodiment, the oral dosage form of the present invention is an extended release dosage form of the active agent dabigatran etexilate.

One possibility for achieving the modified release of the active substance is based on the embedding of the active substance in a matrix which is formed from a polymer. There is a multiplicity of known polymers which can be used for forming such a matrix.

The matrix forming polymer being suitable as release controlling agent is either an erodible or a non-erodible material.

In a first embodiment, the erodible and/or non-erodible material can be described as providing a scaffold (matrix) for embedding the active ingredient and to form a physical barrier, which hinders the active ingredient from being released immediately from the dosage form. Thus, the erodible and/or non-erodible material has the effect that the active ingredient can be released from the scaffold in continuous manner. Release of the drug from the matrix can further be by dissolution controlled as well as diffusion controlled mechanisms. In this first embodiment the erodible and/or non-erodible material functions as matrix forming material.

In a second embodiment, the erodible and/or non-erodible material can be described as a shell-forming material. Preferably, in that embodiment the oral dosage form is a tablet. The release modifying shell preferably encompasses the drug containing tablet core.

In a third embodiment, the erodible and/or non-erodible material can be described as a release modifying coating in a MUPS.

Generally, (i.e. for all three above described embodiments) the oral dosage form of the present invention comprises an erodible and/or non-erodible material. Erodible and/or non-erodible materials are materials, which are able to provide modified release properties, preferably due to their limited solubility, more preferably due to their limited solubility in aqueous conditions at pH 5.0. Preferably, the erodible and/or non-erodible polymer has a water solubility of less than 33 mg/ml at a temperature of 25 °C at a pH of 5.0, more preferably of less than 22 mg/ml, still more preferably of less than 11 mg/ml, especially from 0.01 to 5 mg/ml. The water-solubility is determined according to the column elution method according to EU Directive (67/548/EEC), Annex V, Chapter A6. The pH value is determined according to Ph.Eur. 6.0, 2.2.3. The pH value of the aqueous medium usually is achieved by addition of HCl (or NaOH), if necessary.

The solubility of the erodible and/or non-erodible material can be pH independent or pH dependent. Both embodiments are preferred.

The erodible and/or non-erodible material can comprise an inert material, a lipid material and/or a hydrophilic material. Examples for an inert erodible and/or non-erodible material are ethylcellulose, methacrylate copolymer, polyamide, polyethylene, and polyvinyl acetate; examples for lipid erodible and/or non-erodible materials are carnauba wax, cetyl alcohol, hydrogenated vegetable oils, microcrystalline waxes, monoglycerides, triglycerides and PEG monostearate; examples for hydrophilic erodible and/or non-erodible materials are alginates, carbopol, gelatin, hydroxypropyl cellulose, hydroxypropyl methylcellulose, methylcellulose, xanthan gum and polyethylene oxide. Furthermore, starch derivatives, such as hydroxypropyl starch, fats, waxes, oils, fatty acids, fatty alcohols or glycerol derivatives, such as glycerol dibehenate and glycerol distearate, carrageenan, chitosan, tannin, sodium alginate, alginic acid, acacia and Shellack can be used. Most preferred for the present invention is hydroxypropyl methylcellulose.

In a preferred embodiment, the erodible and/or non-erodible material is an erodible and/or non-erodible polymer. The erodible and/or non-erodible polymer usually has a weight average molecular weight ranging from 30.000 to 3,000,000 g/mol, preferably from more than 50,000 to 2,500,000 g/mol, more preferably from more than 125,000 to 2,000,000 g/mol, still more preferably from 250,000 to 2,200,000 g/mol, particularly preferred from 400,000 to 1,500,000 g/mol. Furthermore, a 2 % w/w solution of the erodible and/or non-erodible polymer in water at pH 7.0 preferably has a viscosity of more than 2 mPas, more preferably of more than 5 mPas, particularly more than 8 mPas and up to 850 mPas, when measured at 25 °C. The viscosity is determined according to Ph. Eur., 6th edition, Chapter 2.2.10. In the above definition the term "solution" may also refer to a partial solution (in case that the polymer does not dissolve completely in the solution). The weight average molecular weight is preferably determined by gel electrophoresis.

It is further preferred that the erodible and/or non-erodible polymer has a melting temperature of below 220 °C, more preferably of between 25 °C and 200 °C. In a particularly preferred embodiment the melting temperature is between 35 °C and 190 °C. The determination of the melting temperature is carried out according to Ph. Eur., 6^{th} edition, Chapter 2.2.15.

If the erodible and/or non-erodible material is a polymeric material, it preferably can be selected from acrylic polymers or acrylic copolymers such as polymers obtained from acrylic acid and/or methacrylic acid monomers. Other preferred polymers include, but are not limited to, cellulose and cellulose derivatives such as cellulose acetate phthalate (CAP), cellulose acetate succinate, cellulose acetate butyrate, cellulose acetate trimellitate, hydroxypropyl methyl cellulose (HPMC), hydroxypropyl methyl cellulose acetate (HPMCA), hydroxypropyl methyl cellulose phthalate (HPMCP) and cellulose acetate succinate (CAS), methyl cellulose, hydroxypropyl cellulose, hydroxyethyl cellulose, methylhydroxyethyl cellulose, ethylhydroxyethyl cellulose, cellulose, carboxymethylcellulose sodium, hydroxypropylmethyl cellulose acetate succinate, ethyl cellulose, sodiumethyl cellulose sulfate, carboxymethylethyl cellulose sodium, cellulose acetate, polyvinyl polymers such as polyvinylacetate, polyvinylpyrrolidone, polyvinyl alcohol phthalate, polyvinyl acetate phthalate and polyvinyl butyl phthalate, polyethylene oxide, polyethylene glycol and mixtures of one or more of these polymers.

In particular, the following kinds of erodible and/or non-erodible polymers are particularly preferred.
1. Cellulose ether, preferably ethyl cellulose, preferably ethyl cellulose having an average molecular weight of 150,000 to 300,000 g/mol and/or an average degree of substitution, ranging from 1.8 to 3.0, preferably from 2.2 to 2.6. This embodiment preferably is used for MUPS or core/shell-tablets.
2. Cellulose ester, preferably cellulose acetate phthalate, carboxymethyl ethyl cellulose, hydroxypropyl methylcellulose phthalate. This embodiment is preferably used for core/shell tablets.
3. Copolymers of methacrylic acid or methacrylic acid esters, preferably ethylacrylate-methyl methacrylate and methacrylic acid-methylmethacrylate. Particularly preferred are ethylacrylate-methylmethacrylate-trimethylammonioethylmethacrylate-chloride, e.g., Eudragit^{®} RL PO (Röhm) and Eudragit^{®} RS PO (Röhm), poly(methacrylic acid-co-ethyl acrylate), e.g. Eudragit^{®} L (Röhm), poly(methacrylic acid-co-methyl methacrylate), e.g. Eudragit^{®} S (Röhm), poly(ethyl acrylate-co-methyl methacrylate), e.g. Eudragit^{®} NE (Röhm) and Eudragit^{®} NM (Röhm).
4. Polyvinyl acetate or polyvinyl acetate copolymers, preferably polyvinyl acetate phthalate; and mixtures thereof.
5. Hydroxypropyl methylcellulose (HPMC)

Preferred acrylic polymers are, for example, polyacrylate, polymethacrylate as well as derivatives and mixtures or copolymers thereof. The polyacrylates used in the invention preferably show the above indicated parameters (e.g. weight average molecular weight, solubility, etc).

Further, a preferred erodible and/or non-erodible polymer is hydroxypropyl methylcellulose (hypromellose).

The erodible and/or non-erodible material is contained in the tablet in an amount of 5 to 80 wt.%, preferably from 10 to 50 wt.%, most preferably from 15 to 40 wt.%, based upon the total weight of the oral dosage form. If too little erodible and/or non-erodible material is used, the formulations may break up during the passage down the gastrointestinal tract and this, in turn, may lead to a premature release of a large portion of the content of the drug. If too much matrix former is used, there is a risk that some of the drug will be encapsulated and not released from the tablet.

The oral dosage form of the invention further optionally comprises a pore-forming material. The term "channeling agent" is in the art often synonymously used for the pore-forming material of the present invention. Since the pore-forming material is generally soluble in the gastrointestinal tract and leaches out from the oral dosage form, the pore-forming material can be described has having the effect of forming pores, such as small holes within the tablet, through which the active ingredient can be released from the tablet matrix in a controlled manner. Thus, release of the active ingredient generally depends on dissolving the pore forming material and thereby forming a porous matrix of capillaries such that the drug can leach out of the matrix.

The pore-forming substance usually has a water solubility of more than 50 mg/l, preferably more than 100 mg/l, at a temperature of 25 °C and pH 5.0, more preferred of more than 250 mg/l and particularly preferred of more than 25 g/l. The water-solubility of the pore-forming substance may range up to 2.5 kg/l. The water-solubility is determined according to the column elution method in EU Directive 67/548/EEC, Annex V, Chapter A6.

The pore-forming substances can be selected from inorganic substances, preferably from inorganic salts such as NaCl, KCl, Na₂SO₄. Furthermore, the pore-forming substances can be selected from organic substances, in particular from organic substances being solid at 30 °C and having the above-mentioned water solubility. Suitable examples are PEG, particularly PEG, having a weight average molecular weight of from 2,000 to 10,000 g/mol.

Furthermore, polyvinylpyrrolidone, preferably having a weight average molecular weight of from 5,000 to 29,000 g/mol, PEG with a weight average molecular weight of 380 - 4800, polyethylene oxide with a weight average molecular weight of less than 100,000 and a viscosity of less than 20 mPa•s, sugar alcohols like mannitol, sorbitol, xylitol, isomalt, and mono or disaccharides, like lactose, are also suitable as pore-forming substances.

The pore forming material is usually contained in the tablet in an amount of 1 to 50 wt.%, preferably from 2 to 40 wt.%, most preferably from 5 to 30 wt.%, based upon the total weight of the oral dosage form.

As explained above, the present invention concerns three preferred embodiments of the solid oral dosage form. Hence, the present invention further relates to three preferred embodiments of a process for producing said oral dosage forms.

In the first preferred embodiment, the present invention concerns a matrix dosage form, preferably a matrix tablet. The matrix tablet preferably is produced by a process, comprising the steps of
(1-I) providing (and optionally blending) dabigatran etexilate bismesylate, the erodible and/or non-erodbile polymer and optionally a pore forming substance and optionally other excipient(s),
(1-II) optionally agglomerating the components of step (I) to yield granules,
(1-III) compressing the mixture resulting from step (I) or (II) into tablets; and
(1-IV) optionally further coating the tablets.

In a second preferred embodiment of the invention, the oral dosage form is in form of a tablet, comprising a core and a shell.

The tablet of the invention preferably is produced by a process, comprising the steps of
(2-I) mixing dabigatran etexilate bismesylate and optionally a pore forming substance and optionally other excipient(s),
(2-II) optionally agglomerating the components of step (I) to yield granules,
(2-III) compressing the mixture into tablets, and
(2-IV) coating the tablets with a coating comprising the erodible and /or non-erodbile polymer and optionally other excipient(s),
(2-V) optionally, the resulting tablets can further be film-coated.

In the above two embodiments of the invention, the dosage form preferably comprises dabigatran etexilate bismesylate, an erodible and/or non-erodible matrix material, a pore-forming material, a filler, a glidant and a lubricant.

In a further aspect the present invention is related to an osmotic controlled release device comprising dabigatran etexilate bismesylate, preferably in form of a tablet.

The controlled release device comprises:
(A) a core comprising dabigatran etexilate bismesylate and an osmotic agent, and
(B) a water-permeable coating comprising a erodible and/or non-erodible polymer and at least one delivery port.

It is noted that all explanations made above for preferred embodiments (e.g. preferred erodible and/or non-erodible polymers, preferred excipients, preferred ratios and amounts) apply as well for the below described aspect.

In a preferred embodiment of the osmotic controlled release devices the water-permeable, non-dissolving coating, which comprises the erodible and/or non-erodible material surrounding the core, controls the influx of water to the core from an aqueous environment, so as to cause drug release by extrusion of some or all of the core to the environment of use.

The osmotic agent contained in the core of this device may be an aqueous-swellable hydrophilic polymer or it may be an osmogen. The coating is preferably polymeric, aqueous-permeable and has at least one delivery port. Examples of such devices are disclosed more fully in U.S. Patent No. 6,706,283, the disclosure of which is hereby incorporated by reference.

Preferably, the osmotic agent creates a driving force for the transport of water from the environment of use into the core of the device. Exemplary osmotic agents are water-swellable hydrophilic polymers. The amount of water-swellable hydrophilic polymers present in the core may range from about 5 to about 80 wt.%, preferably 10 to 50 wt.%, based on the total weight of the core. Exemplary materials include hydrophilic vinyl and acrylic polymers, polysaccharides such as calcium alginate, polyethylene oxide (PEO), polyethylene glycol (PEG), polypropylene glycol (PPG), poly(2-hydroxyethyl methacrylate), poly(acrylic) acid, poly(methacrylic) acid, polyvinylpyrrolidone (PVP) and cross-linked PVP, polyvinyl alcohol (PVA), PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, hydroxyethyl cellulose (HEC), hydroxypropyl cellulose (HPC), hydroxypropyl methyl cellulose (HPMC), carboxymethyl cellulose (CMC) and carboxyethyl cellulose (CEC), sodium alginate, polycarbophil, gelatin, xanthan gum and sodium starch glycolate. Typical classes of suitable osmotic agents are water-soluble organic acids, salts and sugars that are capable of imbibing water, to thereby effect an osmotic pressure gradient across the barrier of the surrounding coating. Typical useful osmogens include magnesium sulfate, magnesium chloride, calcium chloride, sodium chloride, lithium chloride, potassium sulfate, sodium carbonate, sodium sulfite, lithium sulfate, potassium chloride, sodium sulfate, mannitol, xylitol, urea, sorbitol, sucrose, glucose, fructose, lactose, and mixtures thereof. The core may include a wide variety of additives and excipients that enhance the performance of the dosage form or that promote stability, tabletting or processing.

Such osmotic delivery devices may be fabricated in various geometries including bilayer, wherein the core comprises a drug layer and a sweller layer adjacent to each other; including trilayer, wherein the core comprises a sweller layer "sandwiched" between two drug layers; and including concentric, wherein the core comprises a central sweller composition surrounded by the drug layer.

The coating of the device comprises an erodible and/or non-erodible coating, which preferably is insoluble in water but permeable to water and substantially impermeable to drug and excipients contained therein. The coating preferably contains one or more exit passageways or ports in communication with the drug-containing layer(s) for delivering the drug composition. Preferably, the drug-containing layer(s) of the core contains the drug composition, while the sweller layer consists of an expandable hydrogel, with or without additional osmotic agents. When placed in an aqueous medium, the device imbibes water through the membrane, causing the composition to form a dispensable aqueous composition and causing the hydrogel layer to expand and push against the drug-containing composition, forcing the composition out of the exit passageway. The composition can swell, aiding by forcing the drug out of the passageway. A drug can be delivered from this type of delivery system either dissolved or dispersed in the composition that is expelled from the exit passageway.

In the case of a bilayer geometry, the delivery port(s) or exit passageway(s) may be located on the side of the tablet containing the drug composition or may be located on both sides of the tablet or even on the edge of the tablet so as to connect both the drug layer and the sweller layer with the exterior of the device. The exit passageway(s) may be produced by mechanical means or by laser drilling or by creating a difficult-to-coat region on the tablet by use of special tooling during tablet compression or by other means.

A particularly useful embodiment of an osmotic device comprises: (A) a single-layer compressed core comprising: (i) Dabigatran etexilate bismesylate (ii) a modified cellulose, in particular hydroxyethylcellulose, and (iii) an osmotic agent, wherein the modified cellulose is present in the core from about 2.0% to about 35% by weight and the osmotic agent is present from about 15% to about 70% by weight; a water-permeable layer surrounding the core; and at least one passageway within the layer for delivering the drug to a fluid environment surrounding the tablet.

Several disintegrants tend to form gels as they swell with water, thus hindering the drug delivery from the device. Non-gelling, non-swelling disintegrants provide a more rapid dispersion of the drug particles within the core as water enters the core. Preferred non-gelling, non-swelling disintegrants are resins, preferably ion-exchange resins. A preferred resin is Amberlite™ IRP 88 (available from Rohm and Haas, Philadelphia, PA). When used, the disintegrant is present in amounts ranging from about 1% - 25% of the core composition.

The pharmaceutical preparation according to the present invention can further comprise additional excipients and adjuvants, which are pharmaceutically acceptable. In addition general coating materials might further be comprised in the pharmaceutical preparation, which are preferably applied as a coating to the pharmaceutical preparation of the present invention. Such further excipients and adjuvants are known to the person skilled in the art. In this regard it can be referred to the standard textbook by Fiedler ("Lexikon der Hilfsstoffe für Pharmazie, Kosmetik und angrenzende Gebiete", 5th ed., 2002) and to the "Handbook of Excipients", edited by the American Pharmaceutical Association and Dr. Arthur H. Kibbe, 3rd ed., 2000.

In particular, the pharmaceutical preparation according to the present invention comprises one or more excipients in the form of diluents, binders, disintegrants, glidants, lubricants, flow regulating agents, release agents, and/or coating materials and optionally colorants and/or surfactants.

As diluent one or more components can be used, which contribute part of the dosage form to reach the necessary total mass of the dosage form. Preferable diluents are inorganic phosphates, like dibasic calcium phosphate, or sugars or sugar analogues and derivatives thereof, in particular lactose, such as lactose monohydrate including but not limited to the commercially available tablettose or water-free lactose, dextrose, saccharose, maltodextrin, or sugar alcohols including but not limited to sorbitol, mannitol, isomalt (E953). Cellulose like microcrystalline cellulose or powdered celluloses are also preferable diluents according to the present invention. Diluents can be present in an amount of 0 - 80% by weight, preferably in an amount of 10 - 60% by weight of the total weight of the composition.

As disintegrant one or more components can be used, which decompose the tablet in the gastrointestinal fluid. Preferable disintegrants are microcrystalline cellulose, alginates, pregelatinized starch, modified starch like crosscarmellose, sodium carboxymethyl starch and crosslinked PVP. The disintegrant can be present in an amount of 0 - 20% by weight, preferably in an amount of 1 - 15% by weight of the total weight of the composition.

Additional excipients to be used in the solid pharmaceutical dosage form are binders, which are compounds able to bind together active ingredient and excipients in a mixture. As binder to be used in the pharmaceutical preparation of the present invention polyvinylpyrrolidone (PVP) preferably having the molecular weight of 10,000 to 60,000 g/mol and copolymers of PVP preferably having a molecular weight of 40,000 to 70,000 g/mol, microcrystalline cellulose and hydroxypropylmethyl cellulose are especially preferred.

Additional excipients to be used in the pharmaceutical preparation of the present invention are glidants, which are added to a powder to improve its flowability, such as silicium dioxide or fumed silicium dioxide, and/or amphipatic wetting agents, such as sodium dodecylsulfate (SDS), glycerol monostearate, triglycerides or glycerol behenate. The glidant can be present for example in an amount of 0 - 2% by weight, preferably in an amount of 0.5 - 1.5% by weight of the total weight of the composition.

Optionally, as lubricants can be used in the pharmaceutical preparation of the present invention fatty acids, fatty acid derivates, such as alkali and earth alkali salts of stearic, lauric and/or palmitic acid or talcum. Sodium stearyl fumarate and magnesium stearate are preferred. The lubricant can be present in an amount of 0 - 5% by weight, preferably in an amount of 0.2 - 2% by weight of the total weight of the composition.

A suitable flow regulating agent is for example colloidal silica. The flow regulating agent can be present in an amount of 0 - 8% by weight, preferably in an amount of 0.1 - 3% by weight of the total weight of this composition.

The oral dosage form of the present invention might further comprise one or more of a surfactant as for example sodium lauryl sulfate, sodium dodecyl sulfate (SDS), tocopherole propylenglycole succinate (TPGS), polyoxyethylene sorbitan monooleate (e.g. Polysorbat 80), polyoxylglyceride (e.g. Gelucire^{®}), polyoxyl-40 hydrogenated castor oil (Cremophor^{®}), poloxamer, mono- and diglyceride, fatty acids (e.g. oleic acid, lauric acid), and macrogol. Usually, surfactants can be present in an amount of 0.05 to 2 wt.%, preferably of 0.1 to 1.5 wt.%, based on the total weight of the oral dosage form.

If the dosage form is film coated, suitable film forming excipients are for example cellulose derivates such as hypromellose (HPMC), methyl cellulose, hydroxypropyl cellulose, hydroxyethyl cellulose, methylhydroxyethyl cellulose, ethylhydroxyethyl cellulose, methylhydroxypropyl cellulose and carboxymethyl cellulose sodium, polyvinylpyrrolidone, polyvinylacetate, sugars such as saccharose, glucose, sorbit and lactose, methacrylic acid (acrylate-co)polymers, such as Eudragit^{®} E and polyvinyl alcohol polyethylen glycol copolymer (Kollicoat IR).

In one embodiment of the present invention, the pharmaceutically active agent is released from the pharmaceutical preparation with respect to an IR delivery. In a further embodiment of the present invention, the pharmaceutically active agent is released from the pharmaceutical preparation with respect to an ER delivery.

In a first embodiment of the present invention, the pharmaceutical preparation dissolves more than 50%, preferably more than 75%, most preferably more than 80% of the dabigatran etexilate bismesylate from the preparation not later than 30 minutes from the start of the test using an USP apparatus II (Paddle) using 900 ml of sodium acetate trihydrate buffer (pH 4.5) at 37°C as dissolution medium and a rotation speed of 75 rpm.

In a second embodiment, the pharmaceutical preparation dissolves more than 50 %, preferably more than 75 %, most preferably more than 80 % of the dabigatran etexilate bismesylate from the preparation not later than 15 minutes from the start of the test using an USP apparatus II (Paddle) using 900 ml of sodium acetate trihydrate buffer (pH 4.5) at 37°C as dissolution medium and a rotation speed of 75 rpm.

In a third embodiment, the pharmaceutical preparation dissolves more than 50 %, preferably more than 75 %, most preferably more than 80 % of the dabigatran etexilate bismesylate from the preparation not later than 5 minutes from the start of the test using an USP apparatus II (Paddle) using 900 ml of sodium acetate trihydrate buffer (pH 4.5) at 37°C as dissolution medium and a rotation speed of 75 rpm.

In further preferred embodiments, the features of above first and second, first and third, second and third, and first, second and third embodiments may be combined.

In a fourth embodiment of the present invention, the pharmaceutical preparation dissolves less than 80 %, preferably less than 75 %, most preferably less than 50 % of the dabigatran etexilate bismesylate from the preparation after 30 minutes from the start of the test using an USP apparatus II (Paddle) using 900 ml of sodium acetate trihydrate buffer (pH 4.5) at 37°C as dissolution medium and a rotation speed of 75 rpm.

In a fifth embodiment, the pharmaceutical preparation dissolves less than 80 %, preferably less than 75 %, most preferably less than 60 % of the dabigatran etexilate bismesylate from the preparation after two hours from the start of the test using USP apparatus II (Paddle) using 900 ml of sodium acetate trihydrate buffer (pH 4.5) at 37°C as dissolution medium and a rotation speed of 75 rpm.

In a sixth embodiment, the pharmaceutical preparation dissolves less than 95 % preferably less than 90 %, most preferably less than 80 % of the dabigatran etexilate bismesylate from the preparation after 5 hours from the start of the test using an USP apparatus II (Paddle) using 900 ml of sodium acetate trihydrate buffer (pH 4.5) at 37°C as dissolution medium and a rotation speed of 75 rpm.

In preferred embodiments, the features of the above fourth and fifth, fourth and sixth, fifth and sixth, and fourth, fifth and sixth embodiments may be combined.

The pharmaceutical preparation according to the present invention may be present in the form of a tablet which is coated with one or more coating materials. The cotaing materials are not particularly limited and are known to the person skilled in the art. As far as it is herein referred to a dissolution profile, the dissolution profile is that of an uncoated tablet, if the tablet is not coated, and that of a coated tablet, if the tablet is coated.

A further aspect of the present invention is that the pharmaceutical dosage form can be additionally stabilized when stored in a package.

A particluar advantage of the present invention is a package, which increases the stability of the pharmaceutical preparation as such and under forced degradation conditions. Forced degradation conditions are standardized by the International Conference on Harmonization of Technical Requirements for Registration of Pharmaceuticals for Human Use (ICH).

In one embodiment the present invention relates to a package comprising the pharmaceutical preparation, wherein the packaging material and/or the dessicant stabilizes the pharmaceutical preparation, preferably when stored at a temperature of 40°C and at 75 % RH (relative humidity), or at a temperature of 25°C and at 60 % RH. The package may for example be made of a water-impermeable material, like aluminum or polyethylene. Aluminum for example provides a good barrier to moisture, vapor and gases. The term "package" includes all different packs suitable to pack the pharmaceutical preparation, including tightly closed containers. Preferable packs are aluminum blister packs, aluminum packs, bottles and small cylindricyl containers. Preferably, a water-impermeable pack or tightly closed container, such as a polyethylene container, comprises a dessicant, wherein the dessicant can be selected from the group consisting of silica gel, bentonite clay, molecular sieve and activated carbon, preferably silica gel and molecular sieve, most preferably molecular sieve.

In one embodiment of the present invention, the package is a water-impermeable pack, preferably an aluminum pack, an aluminum blister pack or polyethylene pack.

In a further embodiment the present invention relates to a water-impermeable pack comprising a dessicant.

Furthermore, the present invention relates to the above described pharmaceutical preparation for use as anti-coagulant, in particular for use in the prevention or treatmemt of blood clots in a patient suffering from, or susceptible to, the formation of blood clots.

Furthermore, the present invention relates to a method for the manufacture of the above described pharmaceutical preparation, which method comprises at least one of the following steps:
a) mixing dabigatran etexilate bismesylate and at least one pharmaceutically acceptable excipient;
b) direct compression;
c) dry compaction; and/or
d) wet granulation.

A particular advantage of the present invention is that the pharmaceutical preparation can be prepared without the requirement of difficult and costly production methods, like the pellet layering methods used in the prior art. It is, for example, possible to prepare tablets according to the invention by mixing the ingredients and direct compression of the obtained mixture. Alternatively, the pharmaceutical preparation can be prepared by dry compaction optionally followed by compression of the obtained granulate. In a further alternative embodiment the pharmaceutical preparation can be obtained by a usual wet granulation method optionally followed by compression of the obtained granulate. Wet granulation is preferably carried out at a low water concentration. Thus, the granulation liquid should have a water content of below 10 % by weight of the total weight of the liquid. Preferably, the granulation liquid is essentially water-free, more preferably water-free. The granulation liquid can, for example, be an alcohol.

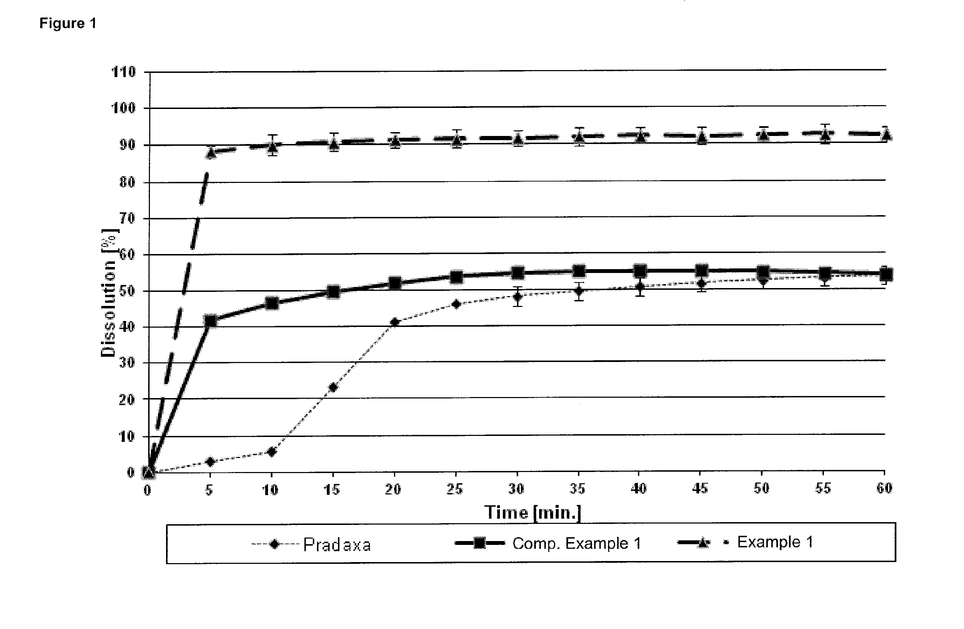

Figure 1 shows the dissolution profiles of the tablets according to example 1, comparative example 1 and Pradaxa^{®}.

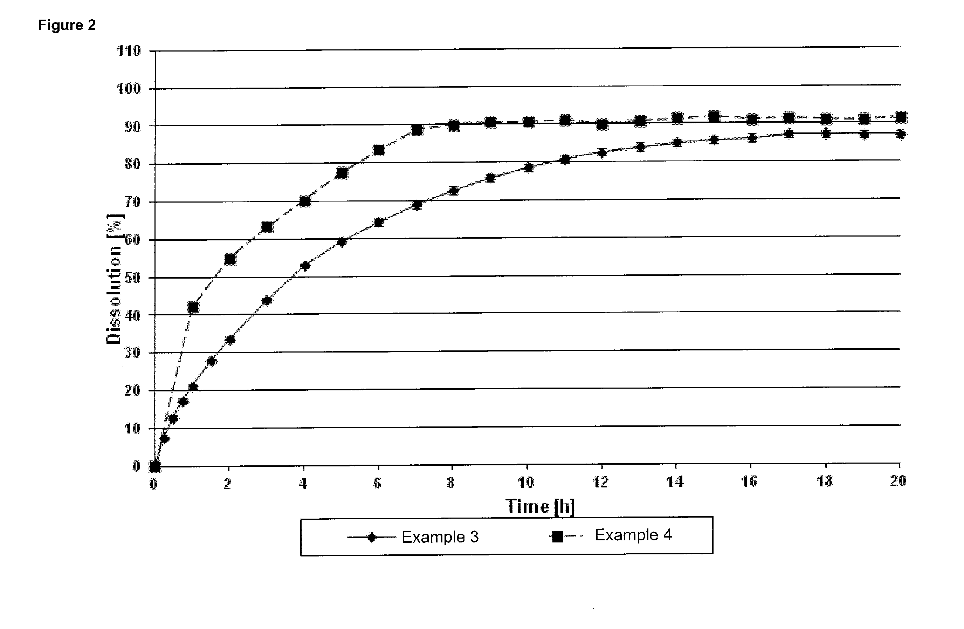

Figure 2 shows the dissolution profiles of the tablets according to examples 3 and 4.

The invention will now be further explained by way of examples which are not to be construed as limiting.

The following analytical methods were used in the examples:

### Chromatographic conditions Assay and RS determination

| | |
|---|---|
| Column: | Phenomenex Luna C18 (2) 100°2050x4,6 5µ, pre-column filter |
| Flow: | 1 ml min⁻¹ |
| Column temp.: | 25 °C |
| Injection volume: | 10 µl |
| Needle wash: | HPLC-grade water |
| Autosampler temp.: | 8 °C |
| Mobile phase: | A: 25 mM KH₂PO₄, pH 6,5 |
| B: | ACN |

### Gradient:

| Time: | % B: |
|---|---|
| 0 | 15 |
| 5 | 48 |
| 10 | 55 |
| 20 | 80 |
| 23 | 80 |
| 25 | 15 |
| 30 | 15 |

| | |
|---|---|
| Run time: | 30min |
| Detector wavelength: | 292 nm, BW 4 nm |
| Reference: | 400 nm, BW 100 nm |
| Slit: | 4 nm |

### Dissolution Parameters (USP app. II)

| | |
|---|---|
| Dissolution medium: | sodium acetate trihydrate buffer pH 4.5 (Example 1, Comparative Example 1 and Pradaxa) or solution having pH 1.2 (Examples 3 and 4) |
| Volume: | 900 ml |
| Temperature: | 37°C ± 0.5°C |
| Agitation: | paddle, 75 rpm |
| Filter: | 1 µm Glass fibre membrane |
| Measurement: | on-line UV/vis measurement |
| Path length: | 1 mm |
| Wavelength: | 292 or 325 nm |
| Background: | subtract average over range, 450 to 500 nm |

### Comparative Examples

Comparative example 1 corresponds to example 2 of WO 2005/018615. Comparative example 2 corresponds to example 3 of WO 2005/018615. Comparative example 3 uses tartaric acid instead of fumaric acid and is otherwise according to example 2 of WO 2005/018615. In comparative example 4, a reduced amount of tartaric acid was employed.

**Table 1**

| | | **Comp. Example 1** | | **Comp. Example 2** | |
|---|---|---|---|---|---|
| | ingredient | amount [mg] | percentage | amount [mg] | percentage |
| 1 | Dabigatran etexilate mesylate | 115.50 | 16.98 | 173.25 | 23.09 |
| 2 | Fumaric acid | 100.00 | 14.70 | 150.00 | 19.99 |
| 3 | Mannitol (Pearlitol^{®} 200SD) | 410.29 | 60.32 | 382.04 | 50.92 |
| 4 | Crospovidone (Kollidon^{®} CL) | 27.20 | 4.00 | 30.00 | 4.00 |
| 5 | Saccharose stearate (Surfhope SE Pharma) | 13.60 | 2.00 | - | - |
| 6 | Magnesium stearate | 13.60 | 2.00 | - | - |
| 7 | Sodium stearylfumarate (PRUV^{®}) | - | - | 15.00 | 2.00 |
| | total | 680.19 | 100.0 | 750.29 | 100.0 |

### Manufacture:

Dabigatran etexilate mesylate was mixed with mannitol and fumaric acid for 15 minutes in Turbula mixer T10B and sieved afterwards. Crospovidone was added and mixed for another 10 min. Magnesium stearate and saccharose stearate or sodium stearylfumarate were added and blended for 5 min. The powder blend was compressed by means of a single punch press Korsch EK0.

**Table 2**

| | | **Comp. Example 3** | | **Comp. Example 4** | |
|---|---|---|---|---|---|
| | ingredient | amount [mg] | percentage | amount [mg] | percentage |
| 1 | Dabigatran etexilate mesylate | 115.50 | 16.98 | 115.50 | 19.11 |
| 2 | tartaric acid | 100.00 | 14.70 | 24.14 | 3.99 |
| 3 | Mannitol (Pearlitol^{®} 200SD) | 410.29 | 60.32 | 410.29 | 67.89 |
| 4 | Crospovidone (Kollidon® CL) | 27.20 | 4.00 | 27.20 | 4.50 |
| 5 | Saccharose stearate (Surfhope SE Pharma) | 13.60 | 2.00 | 13.60 | 2.25 |
| 6 | Magnesium stearate | 13.60 | 2.00 | 13.60 | 2.25 |
| | total | 680.19 | 100.0 | 604.33 | 100.0 |

### Manufacture:

Dabigatran etexilate mesylate is mixed with mannitol and fumaric acid for 15 minutes in Turbula mixer T10B and sieved afterwards. Crospovidone is added and mixed for another 10 min. Magnesium stearate and saccharose stearate are added and blended for 5 min. The powder blend is compressed by means of a single punch press Korsch EK0.

The results of stability testing at accelerated conditions (ICH, Western Europe) are summarized in Table 3.

**Table 3**

| Increase in related substances by HPLC, area-% | 2 weeks 40°C / 75 % RH | 4 weeks 40°C / 75 % RH | 8 weeks 40°C / 75 % RH |
|---|---|---|---|
| **Comparative Example 1** | | | |
| Increase in total impurities HDPE bottle | 1.4% | 4.9% | approx. 15 % |

| **Comparative Example 2** | | | |
|---|---|---|---|
| Increase in total impurities HDPE bottle | 2.4% | 3.9% | 8.7% |

| **Comparative Example 3** | | | |
|---|---|---|---|
| Increase in total impurities HDPE bottle | 2.8% | approx. 10 % | - |

| **Comparative Example 4** | | | |
|---|---|---|---|
| Increase in total impurities HDPE bottle | 5.6 % | approx. 22 % | - |

### Examples according to the invention

Example 1 is describing an immediate release tablet, example 2 an immediate release capsule. Example 3 is an extended release tablet which is a matrix tablet with hypromellose. Also, all other excipients for forming extended release matrix tablets can be used. For forming extended release tablets also a functional coating delaying the dissolution is possible by using this invention. Example 4 is an example for a formulation of a coated tablet.

### Example 1: immediate release tablet

| | ingredient | amount [mg] | percentage |
|---|---|---|---|
| 1 | Dabigatran etexilate bismesylate | 130.62 | 21.94 |
| 2 | Mannitol (Pearlitol 200SD) | 410.29 | 68.92 |
| 3 | Crospovidone (Kollidon CL) | 27.20 | 4.57 |
| 4 | Saccharose stearate (Surfhope SE Pharma) | 13.60 | 2.28 |
| 5 | Magnesium stearate | 13.60 | 2.28 |
| | total | 595.31 | 100.0 |

### Manufacture:

Dabigatran etexilate bismesylate was mixed with mannitol for 15 minutes in Turbula mixer T10B and sieved afterwards. Crospovidone was added and mixed for another 10 min. Magnesium stearate and saccharose stearate were added and blended for 5 min. The powder blend was compressed by means of a single punch press Korsch EK0.

### Example 2: immediate release capsule

| | ingredient | amount [mg] | percentage |
|---|---|---|---|
| 1 | Dabigatran etexilate bismesylate | 130.62 | 56.64 |
| 2 | Mannitol (Pearlitol 200SD) | 99.5 | 43.14 |
| 3 | Silica, highly dispersible (AEROSIL 200) | 0.5 | 0.22 |
| 4 | Hard gelatine capsule, size 1 | - | - |
| | total | 230.62 | 100.0 |

### Manufacture:

Mannitol and sieved silica (mesh size 500 µm) were mixed intensively by means of a Turbula T10B mixer for 1 hour. Sieved dabigatran etexilate bismesylate was added and mixed for another 10 minutes. The powder blend was filled into hard gelatine capsules.

### Example 3: extended release tablet

| | ingredient | amount [mg] | percentage |
|---|---|---|---|
| 1 | Dabigatran etexilate bismesylate | 287.36 | 46.6 |
| 2 | Hypromellose (Methocel K4M Premium DC) | 140.00 | 22.7 |
| 3 | Calcium hydrogenphosphate (Dicafos AN) | 70.00 | 11.35 |
| 4 | Mannitol (Pearlitol 200SD) | 70.00 | 11.35 |
| 5 | Kollidon VA64 | 30.85 | 5.00 |
| 6 | Silica, highly dispersible (AEROSIL 200) | 6.15 | 1.00 |
| 7 | Magnesium stearate | 12.35 | 2.00 |
| | total | 616.71 | 100.0 |

### Manufacture:

The dabigatran etexilate bismesylate was dry compacted and ground. The compacted dabigatran etexilate bismesylate was blended with sieved excipients 2 to 7 for 15 min by means of a Turbula T10B mixer. Magnesium stearate was added and mixed for another 5 minutes. The powder blend was compressed by means of a single punch press Korsch EK0.

### Example 4: extended release tablet

| | ingredient | amount [mg] | percentage |
|---|---|---|---|
| 1 | Dabigatran etexilate bismesylate | 195.9 | 48.5 |
| 2 | Kollidon SR | 195.9 | 48.5 |
| 3 | Silica, highly dispersible (AEROSIL 200) | 4.0 | 1.0 |
| 4 | Magnesium stearate | 8.0 | 2.0 |
| | total | 403.8 | 100.0 |

### Manufacture:

The sieved (mesh size 500 µm) ingredients 1 to 3 were mixed by means of a Turbula T10B mixer for 15 minutes. Magnesium stearate was added and mixed for another 5 minutes. The powder blend was compressed by means of a single punch press Korsch EK0.

### Example 5: extended release coated tablet

| | ingredient | amount [mg] | percentage |
|---|---|---|---|
| 1 | Dabigatran etexilate bismesylate | 130.62 | 23.13 |
| 2 | Calcium hydrogenphosphate (Dicafos^{®} AN) | 195.93 | 34.69 |
| 3 | Mannitol (Pearlitol^{®} 200SD) | 195.93 | 34.69 |
| 4 | Copovidone (Kollidon VA64) | 28.25 | 5.00 |
| 5 | Silica, highly dispersible (AEROSIL^{®} 200) | 2.80 | 0.50 |
| 6 | Sodium stearyl fumarate (PRUV^{®}) | 11.30 | 2.00 |
| | **total** | **564.83** | **100.0** |
| 7 | Eudragit RL 30D (30% dispersion in water) | 94.13 | - |
| 8 | Eudragit RS 30D (30% dispersion in water) | 94.13 | - |
| 9 | Polysorbat 80 | 1.13 | - |
| 10 | Triethylcitrat | 11.30 | - |
| 11 | Glycerolmonostearate | 2.83 | - |
| 12 | Purified Water | 152.86 | |
| | **Total coating dry substance** | **71.74** | |

### Manufacture:

Core: The dabigatran etexilate bismesylate was mixed with silica by means of a Turbula T10B mixer for 30 min. The blended dabigatran etexilate bismesylate was further mixed with sieved excipients 2 to 4 for 15 min. Sodium stearyl fumarate was added and mixed for another 5 minutes. The powder blend was compressed by means of a single punch press Korsch EK0.
Coating: 55% of water was heated to 70-80°C and homogenised for 10 min with 9 to 11. The remaining water was added while stirring with conventional stirrer. The suspension was slowly poured into Eudragit dispersion while gently stirred. The resulting mixture was passed through 0.5 mm sieve and sprayed onto tablets in conventional perforated pan coater (e.g. Lödige LHC25) at inlet air of approx. 35°C.

### Example 6

The results of stability testings at accelerated conditions (ICH, Western Europe) are summarized in Table 4.

**Table 4**

| Increase in related substances by HPLC, area-% | 2 weeks 40°C / 75 % RH | 4 weeks 40°C / 75 % RH | 8 weeks 40°C / 75 % RH |
|---|---|---|---|
| **Example 1** | | | |
| Increase in total impurities HDPE bottle | 1.9% | 2.7% | 4.7% |

| **Example 2** | | | |
|---|---|---|---|
| Increase in total impurities HDPE bottle | 0.8% | 1.2 % | 3.5% |

The results of stability testing at accelerated conditions (ICH, Western Europe) are summarized in Table 5.

**Table 5**

| Increase in related substances by HPLC, area-% | 2 weeks 40°C / 75 % RH | 4 weeks 40°C / 75 % RH |
|---|---|---|
| **Example 3** | | |
| Increase in total impurities HDPE bottle | 1.3% | 1.8% |

## Claims

1. Pharmaceutical preparation comprising dabigatran etexilate bismesylate and at least one pharmaceutically acceptable excipient, **characterized in that** the preparation does not contain any organic acid having a solubility of > 1 g/250 ml of water at 20°C.

2. Pharmaceutical preparation according to claim 1 which does not contain any organic acid exhibiting a pH value in water of below 6 at a concentration of 1 part by weight of the organic acid per 100 parts by weight of water.

3. Pharmaceutical preparation according to claim 1 or 2 which additionally does not contain any inorganic acid exhibiting a pH value in water of below 6 at a concentration of 1 part by weight of the inorganic acid per 100 parts by weight of water.

4. Pharmaceutical preparation according to any of the preceding claims which does not contain any organic or inorganic acid having a pKₐ₁ value below 5 and which preferably does not contain any organic or inorganic acid.

5. Pharmaceutical preparation according to any of the preceding claims which does not contain any acidic excipient

6. Pharmaceutical preparation according to any of the preceding claims, containing 25 to 300 mg of dabigatran etexilate calculated as free base per unit dose based on the total weight of the unit dose.

7. Pharmaceutical preparation according to any of the preceding claims wherein the dabigatran etexilate bismesylate is crystalline.

8. Pharmaceutical preparation according to any of the preceding claims which is an oral solid preparation.

9. Pharmaceutical preparation according to claim 8 which is in the form of a powder mixture, capsule, tablet, pellets or granules.

10. Pharmaceutical preparation according to claim 8 or 9 which is an immediate-release preparation or a modified-release preparation, preferably an extended-release preparation.

11. Pharmaceutical preparation according to any of the preceding claims containing the dabigatran etexilate bismesylate in an extended-release matrix.

12. Pharmaceutical preparation according to any of claims 1-10 wherein the pharmaceutical preparation dissolves more than 50%, preferably more than 75%, most preferably more than 80% of dabigatran etexilate bismesylate from the preparation not later than 30 minutes from the start of the test using an USP apparatus II (Paddle) using 900 ml of sodium acetate trihydrate buffer (pH 4.5) at 37°C as dissolution medium and a rotation speed of 75 rpm.

13. Pharmaceutical preparation according to any of claims 1-11 wherein the pharmaceutical preparation dissolves less than 80 %, preferably less than 75 %, most preferably less than 50 % of dabigatran etexilate bismesylate from the preparation after 30 minutes from the start of the test using an USP apparatus II (Paddle) using 900 ml of sodium acetate trihydrate buffer (pH 4.5) at 37°C as dissolution medium and a rotation speed of 75 rpm.

14. Pharmaceutical preparation according to any of claims 1-13 for use as anti-coagulant.

15. Pharmaceutical preparation according to claim 14 for use in the prevention or treatment of blood clots in a patient suffering from, or susceptible to, the formation of blood clots.

16. Method for the manufacture of a pharmaceutical preparation according to any of the preceding claims, which method comprises at least one of the following steps:
a) mixing dabigatran etexilate bismesylate and at least one pharmaceutically acceptable excipient;
b) direct compression;
c) dry compaction; and/or
d) wet granulation.
